# EUROPÄISCHE PATENTANMELDUNG

(11) **EP 3 702 764 A2**
(43) Veröffentlichungstag der Anmeldung: **02.09.2020**
(21) Anmeldenummer: 20158836.5
(22) Anmeldetag: 21.02.2020
(51) Int. Cl.: G01N 23/02

(54) **PRÜFVORRICHTUNG**

(30) Priorität: 01.03.2019 DE 102019105309
(71) Anmelder: WIPOTEC GmbH, 67657 Kaiserslautern (DE)
(72) Erfinder: Siegrist, Michael, 67659 Kaiserslautern (DE)
(74) Vertreter: Eder Schieschke & Partner mbB

(57) **Zusammenfassung**

Prüfvorrichtung zum Durchstrahlen von Produkten, die der Prüfvorrichtung entlang wenigstens zweier Spuren zugeführt werden, wobei für jede Spur wenigstens ein eigener Sensor vorgesehen ist, um das Erreichen einer vorzugsweise innerhalb eines Gehäuses der Prüfvorrichtung gewählten Sollposition individuell für jede Spur separat überwachen zu können.

## Beschreibung

Die vorliegende Erfindung betrifft eine Prüfvorrichtung zum Durchstrahlen von Produkten und ein Verfahren unter Nutzung einer solchen Vorrichtung.

Bei der industriellen Fertigung von Produkten, insbesondere von Lebensmitteln, ist häufig eine Prüfvorrichtung zur Durchstrahlung der Produkte vorgesehen, um deren ordnungsgemäße Beschaffenheit überprüfen oder Fremdkörper in ihnen entdecken zu können. Hierzu bietet sich beispielweise die Durchstrahlung mit Röntgenstrahlen an. (Andere Durchstrahlungsverfahren sollen hier stellvertretend durch den Begriff "Röntgen" mit erfasst sein). Dabei muss einerseits ein möglichst hoher Produktdurchsatz gewährleistet sein, andererseits muss verhindert werden, dass ein auszuwertender Röntgenstrahl gleichzeitig mehrere Produkte durchstrahlt, etwa weil sich diese entlang des Röntgenstrahls überlagern. Fremdkörper können dann nicht sicher einem der Produkte zugeordnet werden.

Aus Strahlenschutzgründen umfasst die Vorrichtung ein Gehäuse, welches während der Durchstrahlung der im Gehäuse bereitgestellten Produkte gegen den ungewollten Austritt von Röntgenstrahlen abgesichert bzw. verschlossen werden muss, beispielsweise mit einem Schott. Das Schott kann eine Zufuhröffnung im Gehäuse wahlweise verschließen oder freigeben, sodass bei geöffnetem Schott Produkte in das Gehäuse eingebracht werden können. Zweckmäßigerweise werden die Produkte entlang mehrerer, in der Regel parallel zueinander verlaufender Spuren der Prüfvorrichtung zugeführt bzw. durch die Zufuhröffnung in das Gehäuseinnere hineingefördert. Sobald die zur Durchstrahlung vorgesehenen Produkte die Öffnung vollständig passiert haben, kann das Schott geschlossen werden, um mit der Durchstrahlung zu beginnen.

In der insoweit bekannten Praxis kann bei mehrspurig zugeführten Produkten auch der Fall auftreten, dass ein Produkt bereits vollständig innerhalb des Gehäuses angeordnet ist, ein Produkt auf einer anderen Spur die Gehäuseöffnung jedoch noch nicht vollständig passiert hat. Das Schott kann dann nicht geschlossen werden, und das bereits im Gehäuse angeordnete Produkt darf nicht weitergefördert bzw. durchstrahlt werden. Hieraus kann sich eine zeitliche Verzögerung des Produktionsprozesses oder sogar ein Sicherheitsrisiko ergeben, was zu vermeiden ist.

Aufgabe der Erfindung ist es daher, eine Prüfvorrichtung und ein zugehöriges Verfahren anzubieten, welches die vorgenannten Nachteile überwindet.

Die Erfindung beruht auf der Erkenntnis, bei mehrspurig bereitgestellten Produkten, jeder Spur einen eigenen Sensor zuzuordnen, der das Erreichen einer entlang des Förderweges vorgebbaren Sollposition durch das auf der jeweiligen Spur geförderte Produkt detektieren kann. So kann für jede Spur sichergestellt werden, dass ein auf ihr gefördertes Produkt beispielsweise eine im Gehäuse der Prüfvorrichtung festgelegte Sollposition erreicht hat und anschließende Verfahrenshandlungen sicher durchgeführt werden können.

Bei den Produkten kann es sich insbesondere um längliche Nahrungsmittelriegel (Käseblöcke, Wurststränge, etc.) handeln. Grundsätzlich eignen sich die Vorrichtung und das Verfahren aber auch zur Durchstrahlung sonstiger Produkte, die entweder von sich aus formstabil sind oder in geeigneten Behältnissen auf den jeweiligen Spuren transportiert werden können.

Der erfindungsgemäßen Prüfvorrichtung sind solche Produkte entlang wenigstens zweier Spuren, pro Spur hintereinanderliegend, zuführbar, wobei die Spuren bevorzugt parallel zueinander entlang eines Förderweges verlaufen und jede Spur zumindest im Gahäuse über eigene Antriebsmittel verfügt, um das Produkt der jeweiligen Spur individuell fördern zu können. Erfindungsgemäß ist jeder Spur wenigstens ein erster eigener Sensor zugeordnet, um für jede Spur das Erreichen einer entlang des Förderweges vorgebbaren Sollposition durch das auf der jeweiligen Spur geförderte Produkt erfassen zu können. Davon abhängig kann als weiterer Verfahrensschritt dann beispielsweise die Durchstrahlung der Produkte veranlasst werden. Insbesondere sollen die ersten Sensoren Rückschlüsse darauf ermöglichen, ob ein Produkt für das anschließende Durchstrahlen innerhalb des Gehäuses bereitsteht und/oder die Gehäuseöffnung freigegeben hat, sodass das Schott geschlossen werden kann.

Dagegen kann nur ein einzelner, alle Spuren gemeinsam überwachender Sensor (beispielsweise eine quer über die Spuren gerichtete Lichtschranke) nur mit großem Aufwand Hinweise dazu liefern, durch welches Produkt (genauer: auf welcher Spur) die Gehäuseöffnung noch blockiert wird oder eine vordere Sollposition noch nicht erreicht wurde, an der die Durchstrahlung beginnen soll. Durch die erfindungsgemäße Zuordnung individueller Sensoren für jede Spur ist dies jedoch kostengünstig und schnell möglich.

Bevorzugt liegt die von den ersten Sensoren jeder Spur überwachte Sollposition innerhalb des Gehäuses und angrenzend an eine Durchstrahlungszone, in der die Produkte in der zuvor beschriebenen Weise durchstrahlt werden können. Erreicht ein auf einer Spur gefördertes Produkt mit seiner Vorderkante diese Sollposition, so kann bei bekannter Länge des Produktes (in Förderrichtung gemessen) darauf geschlossen werden, ob das rückwärtige Ende des Produktes vollständig durch die Gehäuseöffnung in das Gehäuse hineingefördert wurde. Trifft dies für alle zur Förderung genutzten Spuren zu, haben also alle Produkte die Gehäuseöffnung freigegeben, so kann diese durch das Schott verschlossen werden, um die anschließende Durchstrahlung der Produkte zu beginnen.

Denkbar ist ein Verfahren, bei dem das Produkt jeder Spur zunächst so weit gefördert wird, bis es die Sollposition erreicht bzw. vom zugehörigen Sensor erfasst wird. Sodann wird das Produkt angehalten. Es befindet sich dann unmittelbar an der Sollposition, und - bei bekannter Länge in Förderrichtung - ausreichend weit innerhalb des Gehäuses, um das Schott nicht zu blockieren. In gleicher Weise werden auch die weiteren Spuren betrieben. Nach Beendigung des Verfahrens sind alle vordersten Produkte der für die Förderung verwendeten Spuren an der Sollposition positioniert, und das Schott kann geschlossen werden. (Vorzugsweise kann jedes Produkt nach Erreichen der Sollposition auch geringfügig zurückverfahren werden. Die einzelnen ersten Sensoren werden dadurch freigegeben für weitere Verarbeitungsschritte, bei denen beispielsweise das erneute Passieren dieser und weiterer Sensoren erfasst und in der Verarbeitungssteuerung berücksichtigt wird.)

Zur Durchstrahlung können die Produkte je nach Anforderungen an das Strahlungsergebnis nacheinander oder in Gruppen durch den stromabwärts der Sollposition angeordneten Röntgenstrahl hindurchgefördert werden. Nach Beendigung der Durchstrahlung kann das Gehäuse an einem in Förderrichtung stromabwärts angeordneten Schott wieder geöffnet werden, um die Produkte aus der Prüfvorrichtung herauszufördern. Das eingangsseitige Schott kann ebenfalls wieder geöffnet werden, um weitere Produkte, die auf den einzelnen Spuren bereitzustellen sind, in das Gehäuseinnere hineinzufördern.

Die Sollposition befindet sich vorzugsweise soweit innerhalb des Gehäuses, dass die dort angekommenen Produkte das Schott nicht mehr blockieren. Denkbar ist jedoch auch, die Sollposition näher an das Schott bzw. die Gehäusewand heranzulegen, um anderen Überwachungsanforderungen zu genügen. Auch muss die Sollposition nicht mit der Position des jeweiligen ersten Sensors übereinstimmen. Je nach Art der Sensoren können diese auch eine vom Sensor beabstandete Position überwachen.

Nach einer vorteilhaften Ausführungsform umfasst die Prüfvorrichtung zusätzlich zu den ersten Sensoren pro Spur jeweils wenigstens einen zweiten, eigenen Sensor für jede Spur, um eine von der Sollposition abweichende Prüfposition entlang des Förderweges zu überwachen. Der zweite Sensor jeder Spur kann funktionell oder konstruktiv analog zum ersten Sensor ausgebildet sein. Durch die Anordnung von wenigstens zwei Sensoren entlang einer jeden Spur lässt sich beispielsweise eine Maximal- oder Mindestlänge eines Produktes ohne Abgleich mit einem Encoder überprüfen, was den Ablauf einer Röntgensequenz beschleunigt bzw. optimiert. Zugleich lässt sich feststellen, ob auf ein vorderstes Produkt entlang einer Spur ein weiteres Produkt auf dieser Spur unerwartet oder in zu geringem Abstand nachfolgt, wobei der Abstand durch die Anordnung der ersten und zweiten Sensoren entsprechend definiert bzw. überprüft werden kann.

Auch unbelegte Spuren können erfindungsgemäß durch die individuelle Spurüberwachung leicht erkannt werden, so dass für diese Spur das Erreichen der Sollposition durch ein Produkt nicht abgewartet werden muss. Entsprechend schneller kann die nächste Durchstrahlung erfolgen.

Die Prüfvorrichtung umfasst eine Strahlungsquelle, insbesondere eine Röntgenstrahlungsquelle, um die Produkte durchstrahlen zu können. Die Prüfvorrichtung umfasst außerdem ein Gehäuse, in dem die Strahlungsquelle angeordnet ist und welches gegen den ungewollten Austritt von Röntgenstrahlen in geeigneter Weise abgesichert werden kann. Die wenigstens zwei Spuren, entlang derer Produkte in das Gehäuse der Prüfvorrichtung gefördert werden sollen, verlaufen durch eine verschließbare Zufuhröffnung des Gehäuses in das Innere und führen an anderer Stelle aus dem Gehäuse wieder heraus. Die Sollposition ist dabei innerhalb des Gehäuses angeordnet. Wird die Sollposition auf allen für die Förderung genutzten Spuren von den dortigen Produkten erreicht, wird die Gehäuseöffnung entsprechend als "frei" angenommen und kann durch ein strahlensicheres Schott verschlossen werden, um die Durchstrahlung der Produkte zu beginnen. Ist die Länge der Produkte nicht bekannt, so kann durch wenigstens einen zweiten Sensor, der eine von der Sollposition abweichende Prüfposition überwacht, zumindest sichergestellt werden, dass das Produkt beispielsweise die Prüfposition verlassen hat. Liegt die Prüfposition nahe an der Gehäuseöffnung, beispielsweise knapp innerhalb des Gehäuses, so kann der mindestens eine zweite, oder ein dafür jeweils pro Spur vorgesehener zweiter Sensor die jeweilige Freigabe der Gehäuseöffnung signalisieren, unabhängig davon, ob das jeweilige Produkt bereits die stromabwärts liegende Sollposition erreicht hat. Somit könnte mit dem Schließen des Schotts bereits dann begonnen werden, wenn der mindestens eine zweite oder alle zweiten Sensoren die Freigabe der Gehäuseöffnung signalisieren, während gleichzeitig alle bereits im Gehäuse befindlichen Produkte noch bis an die Sollposition weitergefördert werden. Dies reduziert die Bearbeitungszeit.

Um die Produkte innerhalb des Gehäuses der Prüfvorrichtung fördern zu können, sind dort eigene Antriebsmittel für jede Spur vorgesehen. Zweckmäßigerweise grenzen diese Antriebsmittel, die als Förderbänder, Ketten oder sonstige, dem Fachmann bekannte Transportmittel ausgebildet sein können, innenseitig möglichst nah an die Gehäuseöffnung an, um von außen zugeführte Produkte möglichst einfach übernehmen zu können.

Bevorzugt sieht eine weitere Ausführungsform der Erfindung vor, dass die Prüfvorrichtung auch außerhalb des Gehäuses Antriebsmittel vorsieht. Entlang einer ausgewählten Spur kann dann ein Produkt zunächst über die außerhalb des Gehäuses angeordneten Antriebsmittel an das Gehäuse herangeführt werden. Bei geöffnetem Schott übergeben die äußeren Antriebsmittel das Produkt durch die Zufuhröffnung an die im Gehäuse angeordneten Antriebsmittel derselben Spur, die das Produkt durch Weiterförderung vollständig übernehmen und in das Gehäuse einziehen.

Nach einer Variante der Erfindung sind die außerhalb des Gehäuses vorgesehenen Antriebsmittel als ein einziges, allen Spuren gemeinsam dienendes Transportmittel ausgebildet, etwa als Förderband, dessen Breite sich über alle Spuren erstreckt. Alle Produkte werden dem Gehäuse dann zwar auf individuellen Spuren, aber gemeinsam zugeführt. Durch die erfindungsgemäß separat ausgebildeten Antriebe innerhalb des Gehäuses lassen sich fehlerhafte Positionierungen auf dem Förderband ausgleichen und die Produkte für jede Spur individuell positionieren.

Zweckmäßigerweise sind jedoch sowohl die innerhalb des Gehäuses als auch die außerhalb des Gehäuses angeordneten Antriebsmittel für jede Spur individuell betätigbar, um die Förderung der Produkte unabhängig von benachbarten Spuren durchführen zu können. Diese Lösung erlaubt es auch, ein vorausgehendes Produkt einer Spur in das Gehäuse, beispielsweise bis an die Sollposition hineinzufördern, während unabhängig davon auf der gleichen Spur außerhalb des Gehäuses ein nachfolgendes Produkt bis an das Gehäuse herangefördert werden kann, um für den nächsten Durchstrahlungsvorgang bereitzustehen. Vorzugsweise sind die Antriebsmittel auch so betreibbar, dass das jeweilige Produkt rückwärts, also entgegen der eigentlichen Förderrichtung, transportiert werden kann.

Obwohl jedes Antriebsmittel über einen eigenen Motor betätigbar und steuerbar sein könnte, ist auch eine Variante denkbar, bei der ein Motor für mehrere Spuren bereitsteht, die individuell mit dem Motor (beispielsweise einer Motorwelle) koppelbar und entkoppelbar sind. Dies reduziert den konstruktiven Aufwand und die Kosten.

Nach einer vorteilhaften Ausführungsform der Erfindung umfasst die Prüfvorrichtung auch eine Waage, um das Gewicht der entlang der Spuren transportierten Produkte zu erfassen. Die Waage kann vorteilhafterweise als Mehrspurwaage ausgebildet sein. Allerdings können die Produkte alternativ auch vereinzelt und dann der Waage nacheinander zugeführt beziehungsweise gemeinsam zugeführt und dann einzeln weitergefördert werden (Differenzwägung). Die Waage kann in Förderrichtung gesehen stromaufwärts oder stromabwärts der Durchstrahlung bzw. des Röntgenstrahls angeordnet sein.

Die erfindungsgemäße Prüfvorrichtung umfasst vorzugsweise eine Steuereinheit, um insbesondere die Antriebsmittel der einzelnen Spuren anzusteuern. Auch das vorzugsweise automatisierte Öffnen oder Verschließen der Gehäuseöffnungen und der Betrieb der Röntgenquelle kann durch die Steuereinheit gesteuert werden, die auch zur Erfassung und Auswertung der Signale der einzelnen Sensoren ausgebildet sein sollte. Auch eine in die Prüfvorrichtung integrierte Waage kann über eine solche übergeordnete Steuereinheit angesteuert werden. Natürlich kann die Steuereinheit weitere Schnittstellen zum Datenaustausch bzw. zur Netzwerkanbindung aufweisen.

Das erfindungsgemäße Verfahren zum Betrieb der zuvor beschriebenen Prüfvorrichtung umfasst wenigstens folgende Schritte:
- Zunächst werden Produkte entlang des Förderweges auf wenigstens zwei voneinander unabhängigen Spuren an eine erfindungsgemäße Prüfvorrichtung herangefördert, beispielsweise mittels außerhalb des Gehäuses der Prüfvorrichtung angeordneten, für jede Spur individuell ansteuerbarer Antriebsmittel.
- Entlang des Förderweges ist für jede Spur wenigstens eine Sollposition festgelegt, deren Erreichen durch ein entlang der Spur gefördertes Produkt mit dem für jede Spur separat vorgesehenen ersten Sensor überwacht wird.
- Weiterhin erfindungsgemäß wird die Förderung entlang derjenigen Spur unterbrochen, deren zugeordneter erster Sensor das Erreichen der Sollposition durch das entlang der jeweiligen Spur geförderte Produkt signalisiert,

Dieses Verfahren gestattet die Überwachung der einzelnen Spuren dahingehend, ob ein jeweils dort gefördertes Produkt eine bestimmte Position entlang des Förderweges erreicht hat. Vorzugsweise ist die Sollposition innerhalb des Gehäuses der Prüfvorrichtung und angrenzend an eine Durchstrahlungszone angeordnet. Die Durchführung des Verfahrens kann dann umfassen, dass das vorderste Produkt jeder Spur jeweils bis an die Sollposition herangefördert wird, um dann die jeweilige Förderung zu unterbrechen, und - optional - das Produkt um ein Maß zurückzufahren.

Liegt die Sollposition soweit innerhalb des Gehäuses, dass auch das längste zu erwartende Produkt mit seinem rückwärtigen Ende dann nicht mehr die Gehäuseöffnung durchragt, wenn sein vorderes Ende die Sollposition erreicht hat, genügt dies, um das Gehäuse gefahrlos zu schließen und mit der Durchstrahlungssequenz der Produkte zu beginnen.

Das Verfahren kann weiterhin die Überwachung wenigstens eines zweiten Sensors umfassen, der gemeinsam für alle Spuren oder als eine Gruppe individueller Sensoren pro Spur eine Prüfposition stromaufwärts oder - bevorzugt - stromabwärts der Gehäuseöffnung und damit innerhalb des Gehäuses überwacht. Diese zweiten Sensoren werden vorzugsweise zur Überwachung des rückwärtigen Endes der geförderten Produkte ausgewertet. Die zweiten Sensoren könnten beispielsweise unmittelbar angrenzend an die Gehäuseöffnung im Gehäuseinneren angeordnet sein, wobei jeweils ein zweiter Sensor jeder Spur zugeordnet ist. Erkennt ein solcher Sensor kein Produkt in seinem Erfassungsbereich, kann dies als Indiz dafür ausgewertet werden, dass in der betreffenden Spur kein Produkt durch die Gehäuseöffnung hindurchragt. Gilt dies für alle verwendeten Spuren, so kann das Gehäuse verschlossen werden, und zwar unabhängig davon, ob die Produkte mit ihren vorderen Enden die weiter im Inneren der Prüfvorrichtung definierte Sollposition erreicht haben oder nicht. Die Weiterförderung der Produkte innerhalb der Prüfvorrichtung bis zum jeweiligen Erreichen der Sollposition kann also zeitsparend noch erfolgen, während das Gehäuse bereits verschlossen wird oder verschlossen ist.

Die Durchstrahlung der einzelnen Produkte kann erfolgen, indem das Produkt einer ersten Spur über die Sollposition hinaus in Förderrichtung durch den Röntgenstrahl gefördert wird, während der das Produkt durchstrahlende Röntgenstrahl erfasst und ausgewertet wird. Hat das Produkt der ersten Spur den Röntgenstrahl vollständig passiert, kann das zweite Produkt durch den Röntgenstrahl gefördert werden und so weiter. Wenn die Durchstrahlung und die zugehörige Auswertung es zulässt, können auch mehrere Produkte gleichzeitig durch den vorzugsweise aufgefächerten Röntgenstrahl hindurch gefördert werden. Nach Beendigung der Durchstrahlung aller Produkte können sie die Prüfvorrichtung durch eine weitere Gehäuseöffnung verlassen und einer weiteren Bearbeitungsstation zugeführt werden. Auch die Zufuhröffnung kann durch Entfernen des Schotts wieder freigegeben werden, um die stromaufwärts und außerhalb des Gehäuses bereitstehenden, nachfolgenden Produkte jeder Spur in das Gehäuse hineinzufördern.

Nachfolgend soll eine Ausführungsform einer erfindungsgemäßen Prüfvorrichtung anhand eines Figurenbeispiels näher erläutert werden. Dabei zeigt
- Fig. 1: eine schematische Seitenansicht einer geöffneten Prüfvorrichtung und
- Fig. 2: eine schematische Draufsicht auf die Prüfvorrichtung gemäß Fig. 1.

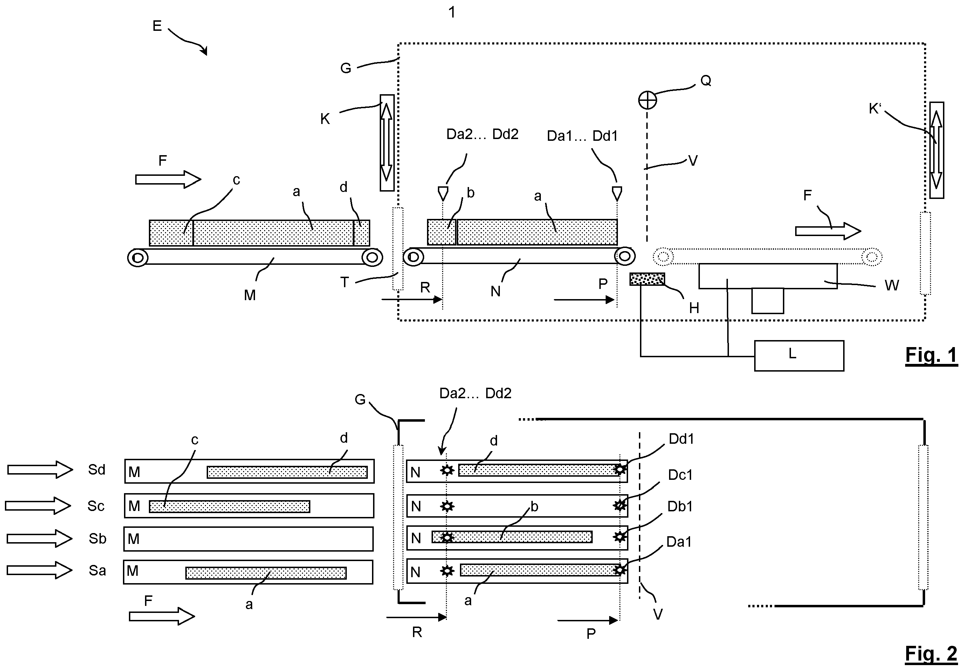

Figur 1 zeigt in einer vereinfachten, seitlichen Schnittdarstellung eine erfindungsgemäße Prüfvorrichtung E. Die Prüfvorrichtung E umfasst ein strahlensicheres Gehäuse G, innerhalb dessen eine Röntgenquelle Q zur Ausgabe eines aufgefächerten Röntgenstrahls V ausgebildet ist. Der Röntgenstrahl V durchstrahlt die Produkte a, b, c, d... wenn sie ihn passieren. Bevorzugt wird die Röntgenquelle Q nur für diese Zeit aktiviert und das Gehäuse G ist dann geschlossen. Die Durchstrahlung wird von einer Erfassungseinheit H erfasst und in umgewandelter Form einer übergeordneten Steuerung L zur weiteren Auswertung zugeführt.

Die Produkte a, b, c, d, ..., werden dem Gehäuse G entlang mehrerer, entlang eines Förderweges F ausgerichteter Spuren Sa, Sb, Sc und Sd zugeführt. Die Spuren liegen in der Ansicht gemäß Figur 1 hintereinander, in der vereinfachten Draufsicht gemäß Figur 2 ist ihre Anordnung nebeneinander zu erkennen. Unter "Spur" muss keine physikalische Komponente verstanden werden, vielmehr soll dieser Begriff einen individuellen Förderweg beschreiben, der konstruktiv durch geeignete Förder- oder Antriebsmittel realisiert ist, zum Beispiel ein Band, ggfls. mit seitlichen Führungselementen. Jede der Spuren Sa, Sb, Sc und Sd umfasst gemäß Figuren 1 und 2 mindestens zwei, jeweils getrennt voneinander ausgebildete Antriebsmittel M, N. Innerhalb des Gehäuses G sind vier als solche Antriebsmittel dienende Förderbänder N dargestellt, die jeweils ein Produkt in Förderrichtung F innerhalb des Gehäuses G fördern können. Außerhalb des Gehäuses G, und jeweils fluchtend mit den innerhalb des Gehäuses G angeordneten Antriebsmitteln N sind separate Förderbänder als Antriebsmittel M für jede Spur vorgesehen, die den Antriebsmitteln N und dem Gehäuse G vorgeschaltet sind. (In vereinfachter Ausführung ist dort nur ein einziges, allen Spuren gemeinsam dienendes Förderband M vorgesehen).Die Antriebsmittel M dienen dazu, einzelne Produkte entlang der jeweiligen Spur dem Gehäuse G in Förderrichtung F zuzuführen und durch eine Zufuhröffnung T dem jeweiligen Antriebsmittel N innerhalb des Gehäuses zu übergeben.(In Förderrichtung an die Antriebsmittel N anschließend können weitere Antriebsmittel im Gehäuse G vorgesehen sein, um die durchstrahlten Produkte gemeinsam oder in jeder Spur individuell weiter bzw. aus dem Gehäuse G herausfördern zu können. Diese sind in Fig. 1 nur angedeutet.

Jedes der Antriebsmittel M, N kann über einen eigenen Motor verfügen. Alternativ ist auch ein für mehrere Antriebsmittel M und/oder N gemeinsam dienender Motor denkbar, der über ansteuerbare Kupplungen den Antrieb der einzelnen Antriebsmittel bewirkt. Die Ansteuerung der Antriebsmittel bzw. ihrer Motoren kann beispielsweise über die Steuereinheit L erfolgen.

Entlang jeder der Spuren ist im Inneren des Gehäuses G an einer Sollposition P für jede Spur ein einzelner Sensor Da1, Db1, Dc1, Dd1 vorgesehen. Die Sollposition P liegt nahe der Position des Röntgenstrahls V. Die ersten Sensoren Da1, Db1, Dc1, Dd1 dienen dazu, dass Erreichen der Sollposition P durch die Vorderkante der einzelnen Produkte a, b, c, d zu erkennen und in Abhängigkeit davon die Weiterförderung des jeweiligen Produktes durch das zugehörige Antriebsmittel N zu unterbrechen. Eine Anordnung von zweiten Sensoren Da2, Db2, Dc2, Dd2 ist nahe der Gehäuseöffnung T im Inneren des Gehäuses G vorgesehen, wobei jeweils ein Sensor einer der Spuren zugeordnet ist. Diese Sensoren dienen insbesondere dazu, das vollständige Einfahren eines Produktes in das Gehäuse G und damit die Freigabe der Öffnung T zu erfassen, um in diesem Falle das Gehäuse G an der Öffnung T mit einem strahlensicheren Schott K verschließen zu können. Die Antriebsmittel M, auf denen nachfolgende, weitere Produkte außerhalb des Gehäuses G herangefördert werden, werden dann entsprechend so gesteuert, dass keines ihrer Produkte gegen das geschlossene Schott K bewegt wird.

Nach dem Schließen des Schotts K kann die Durchstrahlung der einzelnen, bereits im Gehäuse angeordneten Produkte erfolgen, indem diese seriell oder auch teilweise oder vollständig gleichzeitig durch den Röntgenstrahlfächer V hindurch gefördert werden. Nach erfolgter Durchstrahlung können die Produkte (wiederum seriell oder auch gemeinsam) über geeignete Fördermittel durch eine zweites Schott K' hindurch und aus dem Gehäuse G herausgefördert werden. Außerhalb oder innerhalb des Gehäuses G kann ferner eine Waage W vorgesehen sein, um das Gewicht der Produkte a, b, c, d einzeln oder gruppiert zu erfassen. Auch die Waage W kann zum Datenaustausch mit der Steuereinheit L verbunden sein. Die Steuereinheit L kann dazu innerhalb, vorzugsweise außerhalb des Gehäuses G angeordnet sein.

Die Prüfvorrichtung kann erfindungsgemäß folgendermaßen genutzt werden:
Entlang der Spuren Sa, Sb, Sc, Sd werden dem Gehäuse G der Prüfvorrichtung E Produkte a, b, c, d in der Förderrichtung F zugeführt. Die entlang der Spur Sa zugeführten Produkte werden mit "a" bezeichnet, diejenigen der Spur Sb mit "b" und so weiter.

Bei geöffnetem Schott K kann in jeder Spur ein in Förderrichtung F gesehenes vorderstes Produkt durch die Gehäuseöffnung T in das Gehäuse G der Prüfvorrichtung E gefördert werden. Dazu übernehmen die Antriebsmittel N innerhalb des Gehäuses G die von den Antriebsmitteln M jeweils außerhalb des Gehäuses G herangeführten Produkte. Jedes der Antriebsmittel N wird dabei so angesteuert, dass das jeweils geförderte Produkt mit seiner Vorderkante eine von den Sensoren Da1, Db1, Dc1, Dd1 überwachte Sollposition P erreicht und dann vorzugsweise angehalten wird. Mittels der zweiten Sensoren Da2, Db2, Dc2, Dd2 kann überwacht werden, ob das rückwärtige Ende eines jeden Produktes soweit in das Gehäuse G gefördert wurde, dass die Öffnung T freigegeben und für das Schließen mittels Schott K bereit ist. (Erreicht ein Produkt die Sollposition, nachdem das Schott bereits geschlossen wurde, könnte es je nach Verfahrensvorgaben unterbrechungsfrei durch die Sollposition direkt weiter zur Durchstrahlung gefördert werden, um Zeit zu sparen).

In Figur 2 haben die beispielhaft dargestellten Produkte a und d die Sollposition P erreicht. Das Produkt b auf der Spur Sb hat weder die Sollposition P erreicht noch die Prüfposition R freigegeben. Die Auswertung zumindest des Sensors Db2 ergäbe hier, dass das Schließen des Schotts K noch warten muss. Auf der Spur Sc wurde bisher kein Produkt in das Gehäuse G hineingefördert. Das außerhalb des Gehäuses G in der Spur Sc herangeförderte Produkt c kann zu diesem Zweck weitergefördert werden. Alternativ (etwa aus Gründen zeitlicher Optimierung) könnte Spur Sc für den aktuellen Durchstrahlungstakt leer bleiben, sodass das außerhalb des Gehäuses bereitstehende, nächste Produkt c erst für den nächsten Durchstrahlungstakt in das Gehäuse hineingefördert wird.

Sobald alle zur Durchstrahlung vorgesehenen Produkte die vordere Sollposition P erreicht, zumindest aber die Prüfposition R freigegeben haben, kann das Schott K geschlossen werden, um anschließend die Durchstrahlung der Produkte vorzunehmen. Währenddessen können außerhalb des Gehäuses herangeförderte, weitere Produkte durch geeignete Ansteuerung der Antriebsmittel M in Gehäusenähe bereitgestellt werden für den nächsten Durchstrahlungszyklus. Hierzu können weitere, nicht näher dargestellte Sensoren Verwendung finden, welche die Produktförderung der einzelnen Spuren erfasst.

### Bezugszeichen

- a, b, c...: Produkte
- D2, Da2, Db2, Dc2, Dd2...: zweiter Sensor
- Da1, Db1, Dc1, Dd1...: erster Sensor
- E: Prüfvorrichtung
- F: Förderweg
- G: Gehäuse
- H: Erfassungseinheit
- K, K': Schott
- M: zweite Antriebsmittel
- N: erste Antriebsmittel
- P: Sollposition
- Q: Strahlungsquelle
- R: Prüfposition
- Sa, Sb, Sc, Sd...: Spuren
- T: Öffnung
- V: Röntgenstrahl
- W: Waage

## Patentansprüche

1. Prüfvorrichtung zur Durchstrahlung von Produkten (a, b, c, ...), umfassend eine Strahlungsquelle (Q) und ein vor dem Austritt von Strahlung schützendes Gehäuse (G) mit wenigstens einer verschließbaren Öffnung (T),
a) wobei die Produkte (a, b, c, ...) der Prüfvorrichtung zuführbar sind entlang wenigstens zweier Spuren (Sa, Sb, Sc, Sd...), und
b) wobei die Spuren (Sa, Sb, Sc, Sd...) vorzugsweise parallel zueinander entlang eines Förderweges (F) verlaufen und jede Spur im Gehäuse über eigene erste Antriebsmittel (N) verfügt, um das Produkt der jeweiligen Spur individuell fördern zu können,
**dadurch gekennzeichnet,**
c) **dass** jeder Spur wenigstens ein eigener erster Sensor (Da1, Db1, Dc1, Dd1...) zugeordnet ist, um für jede Spur das Erreichen einer entlang des Förderweges (F) im Gehäuse (G) vorgebbaren Sollposition (P) durch das auf der jeweiligen Spur (Sa, Sb, Sc, Sd...) geförderte Produkt (a, b, c...) erfassen zu können.

2. Prüfvorrichtung nach Anspruch 1, **dadurch gekennzeichnet, dass** wenigstens ein weiterer Sensor (D2, Da2, Db2, Dc2, Dd2...) vorgesehen ist, um eine von der Sollposition (P) abweichende Prüfposition (R) entlang des Förderweges (F) zu überwachen.

3. Prüfvorrichtung nach Anspruch 2, **dadurch gekennzeichnet, dass** der wenigstens eine weitere Sensor gebildet wird durch jeweils einen eigenen Sensor (Da2, Db2, Dc2, Dd2...) für jede Spur.

4. Prüfvorrichtung nach Anspruch 2 oder 3, **dadurch gekennzeichnet, dass** auch die Prüfposition (R) innerhalb des Gehäuses (G), vorzugsweise angrenzend an die Öffnung (T), liegt.

5. Prüfvorrichtung nach dem vorhergehenden Anspruch, **dadurch gekennzeichnet, dass** der Abstand zwischen Sollposition (P) und Prüfposition (R) größer gewählt ist als die entlang der Förderrichtung (F) zu messende Länge eines der Produkte (a, b, c...).

6. Prüfvorrichtung nach einem der vorigen Ansprüche, wobei außerhalb des Gehäuses (G) zweite Antriebsmittel (M) vorgesehen sind, die getrennt von den ersten Antriebsmitteln (N) betreibbar sind, um die Produkte der jeweiligen Spur an das Gehäuse (G) heranfördern zu können, wobei die zweiten Antriebsmittel (M) als eine für alle Spuren gemeinsam betreibbare Transporteinheit oder als eine für jede Spur individuell betreibbare Transporteinheit ausgebildet sind.

7. Prüfvorrichtung nach einem der vorigen Ansprüche, ferner enthaltend eine Waage (W) zur Erfassung des Gewichts der Produkte (a, b, c...).

8. Prüfvorrichtung nach einem der vorigen Ansprüche, **dadurch gekennzeichnet, dass** die Antriebsmittel (N) jeder Spur (Sa, Sb, Sc, Sd...) wahlweise mit einem allen Spuren gemeinsamen Antrieb koppelbar und entkoppelbar sind, um die Förderung von Produkten gezielt auf einer Auswahl der Spuren (Sa, Sb, Sc, Sd...) vornehmen zu können.

9. Verfahren zur Durchstrahlung von Produkten (a, b, c...), wobei die Produkte entlang wenigstens zweier Spuren (Sa, Sb, Sc, Sd...) mit einer Prüfvorrichtung nach einem der vorigen Ansprüche bereitgestellt werden, umfassend folgende Schritte:
- Fördern der Produkte (a, b, c...) entlang des Förderwegs (F)
- Überwachen der Sollposition (P) an jeder für die Förderung genutzten Spur (Sa, Sb, Sc, Sd...) mittels des jeweils vorgesehenen erstens Sensors (Da1, Db1, Dc1, Dd1...)
- Unterbrechung der Förderung entlang derjenigen Spur, deren zugeordneter Sensor (Da1, Db1, Dc1, Dd1...) das Erreichen der Sollposition (P) durch ein entlang der jeweiligen Spur (Sa, Sb, Sc, Sd...) gefördertes Produkt (a, b, c...) signalisiert.

10. Verfahren nach Anspruch 9, wobei das Verfahren fortgesetzt wird, bis in jeder für die Förderung genutzten Spur (Sa, Sb, Sc, Sd...) das jeweilige Produkt (a, b, c...) die Sollposition (P) erreicht hat.

11. Verfahren nach einem der vorhergehenden Verfahrensansprüche, **dadurch gekennzeichnet, dass** die Produkte (a, b, c...) nach dem Erreichen der Sollposition (P) soweit entgegen der Förderrichtung (F) zurückverfahren werden, dass sie vom ersten Sensor (Da1, Db1, Dc1, Dd1) nicht mehr erfasst werden und/oder die Sollposition verlassen.

12. Verfahren nach Anspruch 10 oder 11, wobei für alle Spuren wenigstens ein weiterer, zweiter Sensor vorgesehen ist, um das Erreichen oder Verlassen einer zur Sollposition (P) beabstandeten Prüfposition (R) außerhalb oder innerhalb des Gehäuses (G) zu überwachen, wobei als zweiter Sensor entweder ein allen Spuren gemeinsam dienender Sensor (D2) oder ein für jede Spur selbständiger Sensor (Da2, Db2, Dc2, Dd2) vorgesehen ist.

13. Verfahren nach dem vorhergehenden Anspruch, wobei die Auswertung des oder der zweiten Sensoren (D2, Da2, Db2, Dc2, Dd2...) erfolgt zur Ansteuerung eines Schotts (K), welches die Öffnung (T) des Gehäuses verschließen kann, wenn keiner der Sensoren (D2, Da2, Db2, Dc2, Dd2...) ein Produkt (a, b, c...) auf seiner zugeordneten Spur detektiert.

14. Verfahren nach einem der vorigen Verfahrensansprüche, wobei anschließend die Antriebsmittel (N) der zur Förderung genutzten Spuren so angesteuert werden, dass die im Gehäuse positionierten Produkte (a, b, c...) der einzelnen Spuren (Sa, Sb, Sc, Sd...) einzeln oder in Gruppen durch einen Röntgenstrahl (V) gefördert werden.
